# EUROPÄISCHE PATENTSCHRIFT

(11) **EP 0 718 279 B1**
(45) Veröffentlichungstag und Bekanntmachung des Hinweises auf die Patenterteilung: **24.09.1997**
(21) Anmeldenummer: 96101039.4
(22) Anmeldetag: 10.12.1991
(51) Int. Cl.: C07C 253/14, C07C 255/40

(54) **Verfahren zur Herstellung von o-substituierten Benzoylcyaniden**
Process for the preparation of o-substituted benzoylcyanides
Procédé de préparation de benzoylcyanides o-substitués

(30) Priorität: 31.12.1990 DE 4042273; 31.12.1990 DE 4042280; 31.12.1990 DE 4042282; 31.12.1990 DE 4042283; 31.12.1990 DE 4042271; 31.12.1990 DE 4042272
(43) Veröffentlichungstag der Anmeldung: 26.06.1996
(62) Teilanmeldung aus: 91121148.0
(73) Patentinhaber: BASF AKTIENGESELLSCHAFT, 67056 Ludwigshafen (DE)
(72) Erfinder: Wingert, Horst, Dr., D-68159 Mannheim (DE); Wolf, Bernd, Dr., D-67136 Fussgönheim (DE); Benoit, Remy, Dr., D-67435 Neustadt (DE); Sauter, Hubert, Dr., D-68167 Mannheim (DE); Hepp, Michael, Dr., D-68526 Ladenburg (DE); Grammenos, Wassilios, Dr., D-67063 Ludwigshafen (DE); Kükenhöhner, Thomas, Dr., D-67459 Böhl-Iggelheim (DE)

(56) Entgegenhaltungen:
- EP-A- 0 398 782
- EP-A- 0 398 783

## Beschreibung

Die vorliegende Erfindung betrifft ein Verfahren zur Herstellung von o-substituierten Benzoylcyaniden der allgemeinen Formel VI 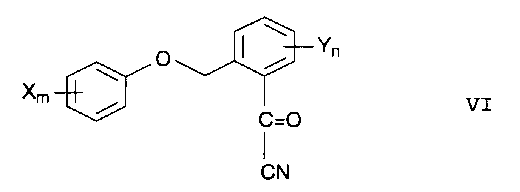 in der die Substituenten und die Indices die folgende Bedeutung haben:
- X,Y: Halogen, C₁-C₄-Alkyl, C₁-C₄-Alkoxy oder Trifluormethyl,
- m: eine ganze Zahl von 0 bis 4, wobei die Reste X verschieden sein können, wenn der Wert von m größer als 1 ist,
- n: eine ganze Zahl von 0 bis 3, wobei die Reste Y verschieden sein können, wenn der Wert von n größer als 1 ist.

Außerdem betrifft die Erfindung neue o-substituierte Benzoylcyanide VI sowie ihre Verwendung als Zwischenprodukte zur Herstellung von 2-Methoxyimino-essigsäureestern der allgemeinen Formel I

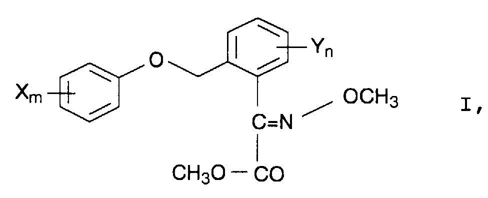

Des weiteren betrifft die Erfindung neue o-substituierte Benzoylchloride der allgemeinen Formel V 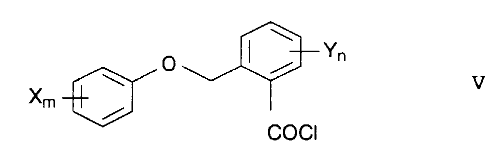 wobei die Variablen die folgende Bedeutung haben:
- X,Y: Halogen, C₁-C₄-Alkyl, C₁-C₄-Alkoxy oder Trifluormethyl,
- m: eine ganze Zahl von 0 bis 4,
- n: eine ganze Zahl von 0 bis 3,
ausgenommen 2-(Phenoxymethyl)-benzoesäurechlorid,
2-(3'-n-Butylphenoxymethyl)-benzoesäurechlorid,
2-(3'-tert.-Butylphenoxymethyl)-benzoesäurechlorid,
2-(3',4'-Dimethylphenoxymethyl)-benzoesäurechlorid,
2-(4'-sec.-Butylphenoxymethyl)-benzoesäurechlorid und
2-(4'-Ethylphenoxymethyl)-4-(methoxy)-benzoesäurechlorid.

Im Einzelnen haben die Variablen die folgende Bedeutung:
X und Y Substituenten, ausgewählt aus einer Gruppe bestehend aus Halogen wie Fluor, Chlor, Brom und Jod, bevorzugt Fluor und Chlor; verzweigten oder unverzweigten C₁-C₄-Alkylgruppen wie Methyl, Ethyl, n-Propyl, Isopropyl, n-Butyl und tert.-Butyl, bevorzugt Methyl und Ethyl; verzweigten oder unverzweigten C₁-C₄-Alkoxygruppen wie Methoxy, Ethoxy, n-Propoxy, Isopropoxy und n-Butoxy, bevorzugt Methoxy, Ethoxy, n-Propoxy und Isopropoxy;
Trifluormethylgruppen;
- m: 0, 1, 2, 3 oder 4, insbesondere 0, 1, 2 oder 3, bevorzugt 0, 1 oder 2;
- n: 0, 1, 2 oder 3, insbesondere 0, 1 oder 2, bevorzugt 0.

Im Hinblick auf die Eigenschaften der Endprodukte sind die folgenden o-substituierten Benzoylcyanide VI besonders bevorzugt:
2-Phenoxymethyl-benzoylcyanid
2-(2'-Chlorophenoxymethyl)-benzoylcyanid
2-(3'-Chlorophenoxymethyl)-benzoylcyanid
2-(4'-Chlorophenoxymethyl)-benzoylcyanid
2-(2',4'-Dichlorophenoxymethyl)-benzoylcyanid
2-(2'-Fluorophenoxymethyl)-benzoylcyanid
2-(3'-Fluorophenoxymethyl)-benzoylcyanid
2-(4'-Fluorophenoxymethyl)-benzoylcyanid
2-(2'-Methylphenoxymethyl)-benzoylcyanid
2-(3'-Methylphenoxymethyl)-benzoylcyanid
2-(4'-Methylphenoxymethyl)-benzoylcyanid
2-(2',4'-Dimethylphenoxymethyl)-benzoylcyanid
2-(2'-Methyl-4'-chlorophenoxymethyl)-benzoylcyanid
2-(2'-Chloro-4'-methylphenoxymethyl)-benzoylcyanid
2-(2'-Methyl-4'-fluorophenoxymethyl)-benzoylcyanid
2-(2'-Ethylphenoxymethyl)-benzoylcyanid
2-(3'-Ethylphenoxymethyl)-benzoylcyanid
2-(4'-Ethylphenoxymethyl)-benzoylcyanid
2-(2'-Ethyl-4'-chlorophenoxymethyl)-benzoylcyanid
2-(2'-Chloro-4'-ethylphenoxymethyl)-benzoylcyanid
2-(2'-Methyl-4'-ethylphenoxymethyl)-benzoylcyanid
2-(2'-Ethyl-4'-methylphenoxymethyl)-benzoylcyanid
2-(2'-Trifluoromethylphenoxymethyl)-benzoylcyanid
3-(3'-Trifluoromethylphenoxymethyl)-benzoylcyanid
2-(4'-Trifluoromethylphenoxymethyl)-benzoylcyanid

Besonders bevorzugte Benzoylchloride V sind:
2-(2'-Chlorophenoxymethyl)-benzoylchlorid
2-(3'-Chlorophenoxymethyl)-benzoylchlorid
2-(4'-Chlorophenoxymethyl)-benzoylchlorid
2-(2',4'-Dichlorophenoxymethyl)-benzoylchlorid
2-(2'-Fluorophenoxymethyl)-benzoylchlorid
2-(3'-Fluorophenoxymethyl)-benzoylchlorid
2-(4'-Fluorophenoxymethyl)-benzoylchlorid
2-(2'-Methylphenoxymethyl)-benzoylchlorid
2-(3'-Methylphenoxymethyl)-benzyolchlorid
2-(4'-Methylphenoxymethyl)-benzoylchlorid
2-(2',4'-Dimethylphenoxymethyl)-benzoylchlorid
2-(2'-Methyl-4'-chlorophenoxymethyl)-benzoylchlorid
2-(2'-Chloro-4'-methylphenoxymethyl)-benzoylchlorid
2-(2'-Methyl-4'-fluorophenoxymethyl)-benzoylchlorid
2-(2'-Ethylphenoxymethyl)-benzoylchlorid
2-(3'-Ethylphenoxymethyl)-benzoylchlorid
2-(4'-Ethylphenoxymethyl)-benzoylchlorid
2-(2'-Ethyl-4'-chlorophenoxymethyl)-benzoylchlorid
2-(2'-Chloro-4'-ethylphenoxymethyl)-benzoylchlorid
2-(2'-Methyl-4'-ethylphenoxymethyl)-benzoylchlorid
2-(2'-Ethyl-4'-methylphenoxymethyl)-benzoylchlorid
2-(2'-Trifluoromethylphenoxymethyl)-benzoylchlorid
3 -(3'-Trifluoromethylphenoxymethyl)-benzoylchlorid
2-(4'-Trifluoromethylphenoxymethyl)-benzoylchlorid.

Benzoylcyanide sind allgemein bekannt, z.B. aus Angew. Chemie 94, 1 (1982).

Die Benzoylcyanide VI sind auf verschiedene Weise erhältlich. Üblicherweise werden sie aus den entsprechenden Säurechloriden und cyanidhaltigen Salzen wie Quecksilbercyanid [Liebig's Annalen 3, 249 (1832)], Kupfercyanid [Organic Synthesis Coll. Vol. III, 112 (1955); Angew. Chemie 94(1), 1 (1982)] und insbesondere Natriumcyanid unter Verwendung eines wäßrig-organischen Zweiphasensystems und in Gegenwart eines Phasentransferkatalysators [Tetrahedron Letters, 2275 (1974)] hergestellt:

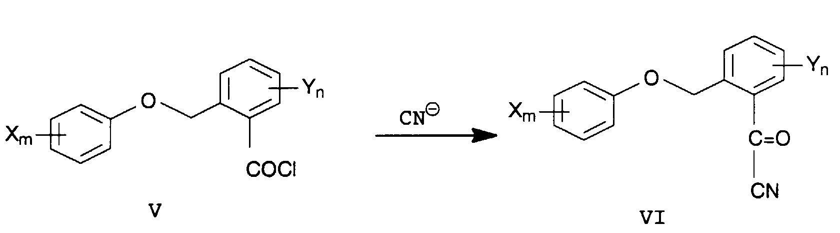

Die Umsetzung erfolgt normalerweise in einem inerten Lösungsmittel oder Lösungsmittelgemisch, wobei das cyanidhaltige Salz bevorzugt in Wasser gelöst und das Säurechlorid V in einem mit Wasser mischbaren oder unmischbaren Lösungsmittel gelöst vorliegt.

Besonders bevorzugt arbeitet man in einem Zweiphasensystem in Gegenwart eines Phasentransferkatalysators, z.B. einem quartären Ammonium- oder Phosphoniumsalz wie Tetrabutylammoniumchlorid, Tetrabutylammoniumbromid, Methyltributylammoniumjodid, Tetrabutylammoniumhydrogensulfat, Trimethylbenzylammoniumchlorid, Triethylbenzylammoniumchlorid, Triphenylbenzylammoniumchlorid und Benzyltributylphosphoniumbromid.

Als cyanidhaltige Salze eignen sich bevorzugt Alkalimetallcyanide wie Lithium-, Natrium- und Kaliumcyanid oder Erdalkalimetallcyanide wie Magnesium-, Calcium- oder Bariumcyanid, insbesondere Natrium- und Kaliumcyanid. Es können aber auch Mischungen der genannten Salze mit Blausäure verwendet werden.

Normalerweise werden das cyanidhaltige Salz oder die Mischung eines cyanidhaltigen Salzes in Blausäure und das Säurechlorid V in stöchiometrischen Mengen eingesetzt, jedoch kann auch ein Überschuß an cyanidhaltigem Salz und gegebenenfalls Blausäure, bis etwa 100 mol-%, vorteilhaft sein.

Die Menge an Phasentransferkatalysator ist nicht kritisch; üblicherweise verwendet man 0,001 bis 1 mol-%, insbesondere 0,01 bis 5 mol-% an Katalysator, bezogen auf die Menge an V.

Zweckmäßigerweise arbeitet man bei Normaldruck. Höherer oder niedrigerer Druck sind möglich, bringen jedoch im allgemeinen keine Vorteile.

Die Reaktionstemperatur liegt im allgemeinen zwischen 0°C und der Siedetemperatur des jeweiligen Lösungsmittel(gemisches), insbesondere zwischen 0 und 40°C.

Die Aufarbeitung des Reaktionsgemisches erfolgt nach an sich bekannten Methoden, so daß sich nähere Ausführungen hierzu erübrigen.

Die Umsetzung kann sowohl diskontinuierlich als auch kontinuierlich durchgeführt werden. Bei der kontinuierlichen Arbeitsweise leidet man die Reaktionspartner beispielsweise durch einen Rohrreaktor oder über Rührkesselkaskaden.

Eine bevorzugte Ausführungsvariante besteht darin, das cyanidhaltige Salz oder die Mischungen eines cyanidhaltigen Salzes in Blausäure zusammen mit dem Phasentransferkatalysator vorzulegen und eine Lösung des Säurechlorids unter intensivem Rühren zuzugeben.

2-(Phenoxymethyl)-benzoesäurechlorid (V, m, n = 0) ist aus der DE-A 1 279 682 bekannt.

Des weiteren sind aus der JP-OS 80/124 777 einige durch Alkylgruppen substituierte 2-(Phenoxymethyl)-benzoesäurechloride als Zwischenprodukte für 6,11-Dihydro-11-oxodibenz(b,e)oxepine bekannt.

Die o-substituierten Benzoylchloride der allgemeinen Formel V 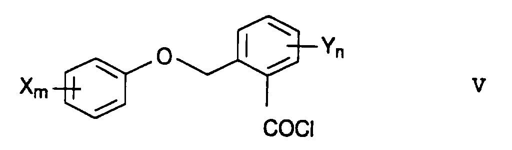 wobei die Variablen die folgende Bedeutung haben:
- X,Y: Halogen, C₁-C₄-Alkyl, C₁-C₄-Alkoxy oder Trifluormethyl,
- m: eine ganze Zahl von 0 bis 4,
- n: eine ganze Zahl von 0 bis 3,
ausgenommen 2-(Phenoxymethyl)-benzoesäurechlorid,
2-(3'-n-Butylphenoxymethyl)-benzoesäurechlorid,
2-(3'-tert.-Butylphenoxymethyl)-benzoesäurechlorid,
2-(3',4'-Dimethylphenoxymethyl)-benzoesäurechlorid,
2-(4'-sec.-Butylphenoxymethyl)-benzoesäurechlorid und
2-(4'-Ethylphenoxymethyl)-4-(methoxy)-benzoesäurechlorid.

Sie können nach an sich bekannten Verfahren durch Behandlung der entsprechenden Carbonsäuren IV mit Phosgen oder Thionylchlorid, zweckmäßigerweise in einem inerten Lösungs- oder Verdünnungsmittel, erhalten werden (vgl. Organikum, VEB Deutscher Verlag der Wissenschaften, 16. Auflage, Berlin 1986, Seiten 423 f).

Als Lösungs- oder Verdünnungsmittel eignen sich beispielsweise aliphatische Kohlenwasserstoffe wie n-Pentan, n-Hexan, Cyclohexan, Petrolether, Weißöle und Ligroin, aromatische Kohlenwasserstoffe wie Benzol, Toluol und o-, m-, p-Xylol, halogenierte Kohlenwasserstoffe wie Methylenchlorid, Chloroform, Tetrachlorkohlenstoff, 1,2-Dichlorethan, 1,1,2-Trichlorethan, 1,1,2,2-Tetrachlorethan, Perchlorethan und Chlorbenzol, Ether wie Diethylether, Tetrahydrofuran und Dioxan, Ester wie Ethylacetat sowie Aceton, Methylketon und Cyclohexanon.

Carbonsäure IV und Chlorierungsmittel werden normalerweise in stöchiometrischen Mengen eingesetzt, bevorzugt verwendet man einen Überschuß an Chlorierungsmittel bis zu 100 mol-%.

Vorteilhaft arbeitet man in Anwesenheit eines Katalysators, beispielsweise eines Amides wie Dimethylformamid, Diethylformamid und Diisobutylformamid, eines tertiären Amins wie Dimethylaminopyridin, Diethylaminopyridin, Morpholinoformiat und 4-Pyrrolidinopyridin oder eines Phosphinoxides wie Tributylphosphinoxid.

Die Menge an Katalysator kann in weiten Bereichen variiert werden. Üblicherweise verwendet man 0,01 bis 50 mol-%, insbesondere 0,1 bis 10 mol-% an Katalysator, bezogen auf die Menge an Säure IV.

Die Reaktionstemperatur ist nicht kritisch. Im allgemeinen arbeitet man zwischen 0 und 100°C, insbesondere bei der Siedetemperatur des jeweiligen Lösungsmittels.

Zweckmäßigerweise erfolgt auch die Herstellung der Säurechloride unter Normaldruck. Höherer oder niedriger Druck sind möglich, bringen jedoch im allgemeinen keine Vorteile.

Die Aufarbeitung des Reaktionsgemisches erfolgt wie üblich.

Eine Verfahrensvariante zur Herstellung der Benzoylcyanide VI besteht darin, die Benzoylchloride V zu synthetisieren und sie ohne Isolierung aus der Reaktionsmischung oder als Rohprodukte mit Alkalimetall- oder Erdalkalimetallcaniden, gewünschtenfalls in Gegenwart von Blausäure, zu behandeln.

Die Carbonsäuren IV sind entweder aus den Druckschriften US-A 3 420 851, JP-OS 80/124 777 oder aus Coll. Czech. Chem. Commun. 32, 3448 (1967) bekannt oder nach den dort beschriebenen Methoden herstellbar. Besonders vorteilhaft stellt man sie durch Umsetzung von Phenolen der Formel II mit Lactonen der Formel III, bevorzugt unter basischen Reaktionsbedingungen, her. 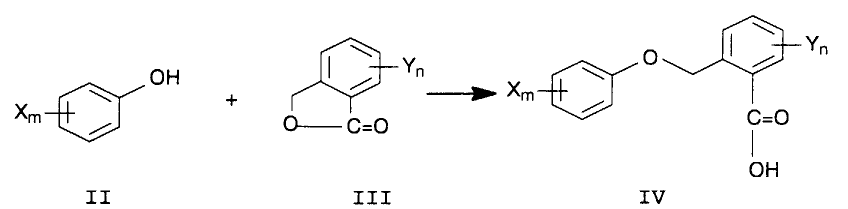 Die o-substituierten Benzoylcyanide VI sind wertvolle zwischen-produkte für die Synthese von E-Oximethern von Phenylglyoxylsäureestern I 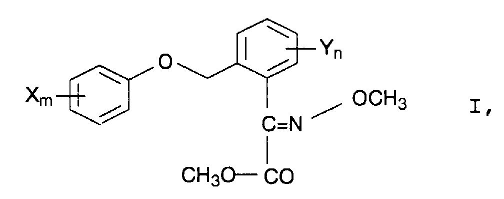 die im Pflanzenschutz vorzugsweise als Fungizide Verwendung finden (vgl. EP-A 253 213 und EP-A 254 426).

Zur Herstellung der Endprodukte I werden die o-substituierten Benzoylcyanide VI, entweder in isolierter Form, als Rohprodukte oder noch in dem bei ihrer Herstellung verwendeten Lösungsmittel gelöst, mit niederen Alkoholen wie Methanol nach der Pinner-Reaktion (vgl. Angew. Chemie 94, 1 (1982) umgesetzt. Dabei erhält man sowohl Phenylgyloxylsäureester VIIa als auch die Ketal der Phenylglyoxylsäureester VIIb. Anschließend setzt man die Verbindungen VIIa oder VIIb oder in Gemisch aus beiden mit O-Methylhydroxylamin oder einem seiner Säureadditonssalze um wobei das hierbei erhaltene E/Z-Isomerengemisch zur weitgehenden Umlagerung der Z-in die E-Isomeren gleichzeitig oder anschließend mit einer Säure behandelt wird.

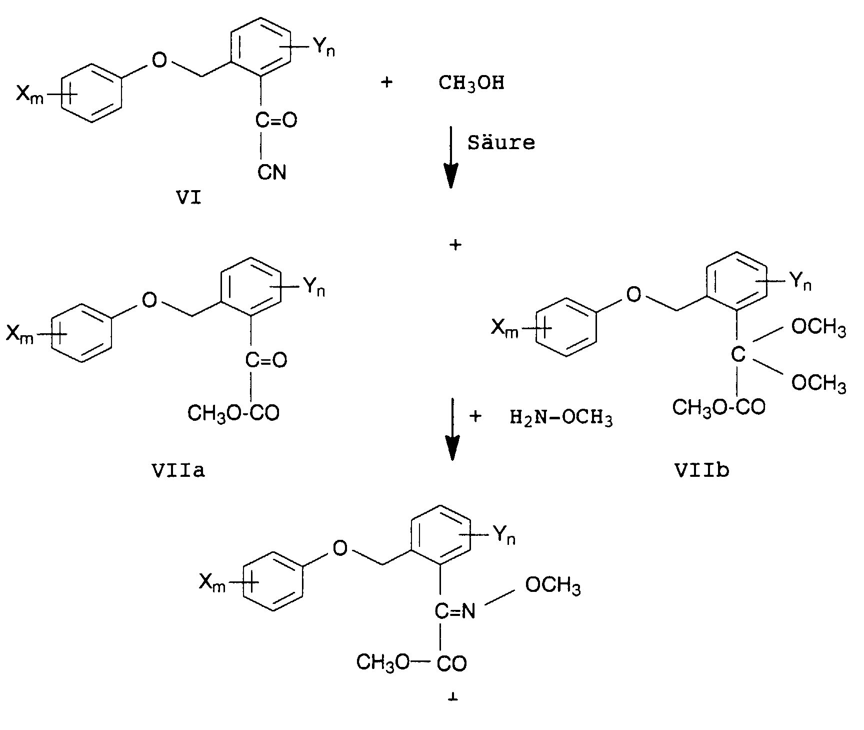

### Beispiele

### Beispiel 1

### 2-Phenoxymethylbenzoylchlorid

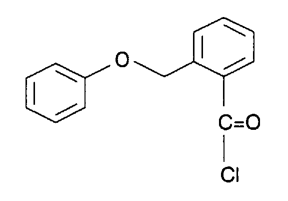

Eine Mischung aus 72 g (0,32 mol) 2-Phenoxymethylbenzoesäure, 56 g (0,47 mol) Thionylchlorid und 300 ml 1,2-Dichlorethan wurde 3 Stunden auf Rückflußtemperatur erhitzt. Nach Entfernen der flüchtigen Bestandteile im Vakuum erhielt man ein dunkles Öl, das langsam auskristallisierte.
Ausbeute: 79 g.
¹-NMR (in CDCl₃, TMS als interner Standard): 5,35 ppm (s,2H); 6,95 ppm (m, 3H); 7,3 ppm (t,2H); 7,5 ppm (t,1H); 7,7 ppm (t,1H); 7,85 ppm (d,1H); 8,35 ppm (d,1H).

### Beispiel 2

### 2-Phenoxymethylbenzoylcyanid

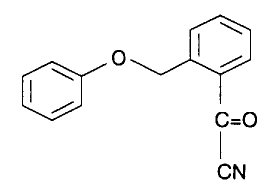

Bei 20°C wurde eine Mischung aus 79 g (0,32 mol) 2-Phenoxymethylbenzoylchlorid, 17 g (0,347 mol) Natriumcyanid, 0,3 g (0,93 mmol) Tetrabutylammoniumbromid, 200 ml Wasser und 300 ml 1,2-Dichlorethan 2 Stunden gerührt. Anschließend trennte man die Phasen und extrahierte die wäßrige Phase mit 100 ml 1,2-Dichlorethan. Die vereinigten organischen Phasen wurden dreimal mit je 100 ml Wasser gewaschen und dann getrocknet. Nach Entfernen des Lösungsmittels unter reduziertem Druck erhielt man 70,8 g eines bräunlichen Öles, das beim Anreiben mit Methyl-tert.-butylether/Pentan(1:1) kristallisierte.

¹H-NMR (in CDCl₃, TMS als inerter Standard): 8,40 ppm (d, 1H); 8,00 ppm (d,1H); 7,80 (ppm (t,1H); 7,60 ppm (t,1H); 7,35 ppm (t,2H); 7,00 ppm (m,3H); 5,45 ppm (s,2H).

### Beispiel 3

2-(2'-Methylphenoxymethyl)benzoylcyanid

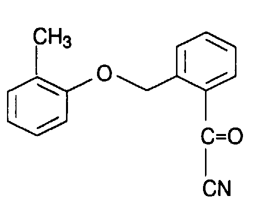

### Variante A:

Zu einer Lösung von 458 g (1,76 mol) 2-(2'-Methylphenoxymethyl)-benzoylchlorid in 2 l Toluol wurden 890 mg (2,8 mmol) Tetra-n-butylammoniumbromid und anschließend eine Lösung von 94,8 g (1,93 mol) Natriumcyanid in 1 l Wasser gegeben. Das Gemisch wurde 2 Stunden bei etwa 20°C gerührt, und dann 14 Stunden stehen gelassen. Anschließend wurde die organische Phase abgetrennt, über Natriumsulfat getrocknet und bei reduziertem Druck eingeengt. Das braune Rohprodukt mit einer Reinheit von 91,7 Gew.-% (nach GC-Analyse) wurde aus 2 l Methyl-tert.-Butylether umkristallisiert.
Ausbeute: 61 %;

### Variante B:

Zu einer Mischung aus 48,2 g (0,98 mol) Natriumcyanid, 2,5 g (7,5 mmol) Tetra-n-butylammoniumchlorid und 640 ml Wasser wurde bei 22°C innerhalb einer Stunde eine Lösung von 231,8 g (0,89 mol) 2-(2'-Methylphenoxymethyl)-benzoylchlorid in 1,55 l Toluol zugetropft. Man rührte noch 14 Stunden, wonach die organische Phase abgetrennt, einmal mit 0,5 l Wasser gewaschen und bei reduziertem Druck eingeengt wurde. Das Rohprodukt wurde aus 0,4 l Cyclohexan umkristallisiert.
Ausbeute: 67 %; Fp.: 74°C (gelb-brauner Feststoff)

### Variante C (Reaktionsführung in Gegenwart von Blausäure)

Zu einer Lösung von 117,4 g (2,4 mol) Natriumcyanid in 725 ml Wasser wurden bei 20°C 38 g einer 19 gew.-%igen wäßrigen Salzsäure (= 0,2 mol HCl) getropft. Nach Zugabe von 1,4 g (4,3 mmol) Tetrabutylammoniumbromid und 2,4 1 Toluol wurde das Gemisch auf 25°C erwärmt und unter intensivem Rühren innerhalb von 15 Minuten mit einer Lösung von 521 g (2,0 mol) 2-(2'-Methylphenoxymethyl)-benzoylchlorid in 710 ml Toluol versetzt. Man rührte noch 90 Minuten, wobei sich die Reaktionsmischung bis 32°C erwärmte. Anschließend wurde die organische Phase abgetrennt, mit 200 ml Wasser und 200 ml 1 gew.-%iger wäßriger Salzsäure gewaschen und dann unter reduziertem Druck eingeengt. Das blaßgelbe Rohprodukt enthielt 93 mol-% 2-(2'-Methylphenoxymethyl)-benzoylcyanid (nach HPLC-Analyse).
Ausbeute: 92 %
¹H-NMR (in CDCl₃, TMS als interner Standard): 2,3 ppm (s,3H); 5,4 ppm (2, 2H); 6,7-7,0 ppm (m,2H); 7,1-7,3 ppm (m, 2H); 7,6 ppm (t,1H); 7,8 ppm (t,1H); 8,0 ppm (d,1H); 8,3 ppm (d,1H);
IR-Spektrum [cm⁻¹]: 2220, 1665, 1600, 1573, 1496, 1242, 1131, 1060, 846, 724.

### Beispiel 4

2-(2'-Methylphenoxymethyl)benzoylchlorid

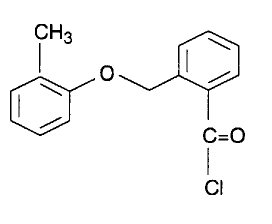

Zu einer Lösung von 435 g (1,80 mol) 2-(2'-Methylphenoxymethyl)-benzoesäure in 1,8 l Toluol wurden 20 bis 25°C 235,6 g (1,98 mol) Thionylchlorid getropft. Die erhaltene Mischung wurde auf 70°C erhitzt, bis keine Gasentwicklung mehr zu beobachten war (ca. 5 Stunden) und dann eingeengt. Das erhaltene braune Öl (Gehalt an Produkt: 92,5 %) kristallisierte bei Abkühlung.
Ausbeute: 93,5 %; Fp.: 48-51°C.
¹H-NMR (in CDCl₃; TMS als interner Standard): 2,3 ppm (s,3H); 5,3 ppm (s,2H); 6,7-6,9 ppm (m,2H); 7,0-7,2 ppm (m,2H); 7,4 ppm (t,1H); 7,6 ppm (t,1H); 7,9 ppm (d,1H); 8,3 ppm (d,1H);
IR-Spektrum [cm⁻¹]: 1737, 1495, 1243, 1026, 1185, 1124, 886, 743, 716, 673.

## Patentansprüche

1. Verfahren zur Herstellung von o-substituierten Benzoylcyaniden der allgemeinen Formel VI 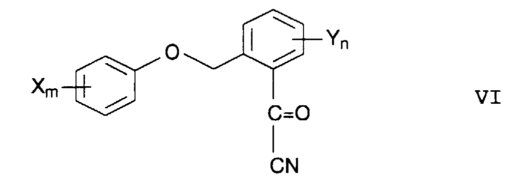 in der die Substituenten und die indices die folgende Bedeutung haben:
X,Y Halogen, C₁-C₄-Alkyl, C₁-C₄-Alkoxy oder Trifluormethyl,
m eine ganze Zahl von 0 bis 4, wobei die Reste X verschieden sein können, wenn der Wert von m größer als 1 ist,
n eine ganze Zahl von 0 bis 3, wobei die Reste Y verschieden sein können, wenn der Wert von n größer als 1 ist,
dadurch gekennzeichnet, daß man eine 2-Phenoxymethylbenzoesäure der allgemeinen Formel IV 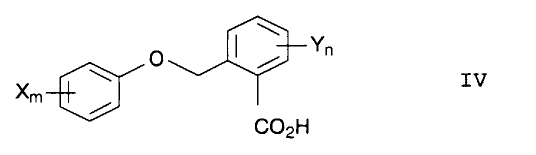 mit Phosgen oder Thionylchlorid in das entsprechende o-substituierte Benzoylchlorid der allgemeinen Formel V 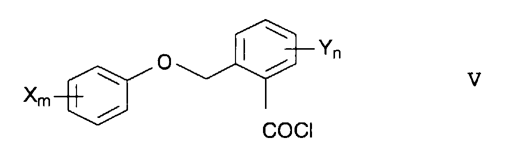 überführt und V anschließend mit einem Alkalimetall- oder Erdalkalimetallcyanid umsetzt.

2. Verfahren nach Anspruch 1, dadurch gekennzeichnet, daß man die Umsetzung von IV nach V bei Temperaturen von 0°C bis 100°C durchführt.

3. Verfahren nach Anspruch 1, dadurch gekennzeichnet, daß man die Umsetzung von IV nach V in Gegenwart eines Katalysators durchführt.

4. Verfahren nach Anspruch 3, dadurch gekennzeichnet, daß man als Katalysator ein Amid oder ein Phoshinoxid verwendet.

5. Verfahren nach Anspruch 1, dadurch gekennzeichnet, daß man die Umsetzung von V nach VI in Anwesenheit von Blausäure durchführt.

6. Verfahren nach Anspruch 1, dadurch gekennzeichnet, daß man die Umsetzung von V nach VI in einem Zweiphasensystem in Gegenwart eines Phasentransferkatalysators durchführt.

7. Verfahren nach Anspruch 1, dadurch gekennzeichnet, daß man die Umsetzung von V nach VI bei Temperaturen von 0°C bis zum Siedepunkt des verwendeten Lösungsmittels bzw. Lösungsmittelgemischs durchführt.

8. Verfahren nach Anspruch 1, dadurch gekennzeichnet, daß man V ohne weitere Isolierung (in situ) zu VI umsetzt.

9. O-substituierte Benzoylchloride der allgemeinen Formel V gemäß Anspruch 1, ausgenommen
- 2-(Phenoxymethyl)-benzoesäurechlorid,
- 2-(3'-n-Butylphenoxymethyl)-benzoesäurechlorid,
- 2-(3'-tert.-Butylphenoxymethyl)-benzoesäurechlorid,
- 2-(3',4'-Dimethylphenoxymethyl)-benzoesäurechlorid,
- 2-(4'-sec.-Butylphenoxymethyl)-benzoesäurechlorid und
- 2-(4'-Ethylphenoxymethyl)-4-methoxybenzoesäurechlorid.

10. 2-(2'-Methylphenoxymethyl)-benzoesäurechlorid.

11. O-substituierte Benzoylcyanide der allgemeinen Formel VI gemäß Anspruch 1.

12. 2-(2'-Methylphenoxymethyl)-benzoylcyanid.

## Claims

1. A process for preparing o-substituted benzoyl cyanides of the formula VI 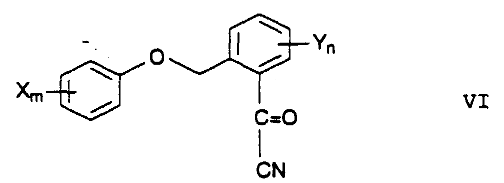 where the substituents and the indices have the following meanings:
X, Y halogen, C₁-C₄-alkyl, C₁-C₄-alkoxy or trifluoromethyl,
m an integer from 0 to 4, it being possible for the radicals X to be different when the value of m is greater than 1,
n an integer from 0 to 3, it being possible for the radicals Y to be different when the value of n is greater than 1,
which comprises converting a 2-phenoxymethylbenzoic acid of the formula IV 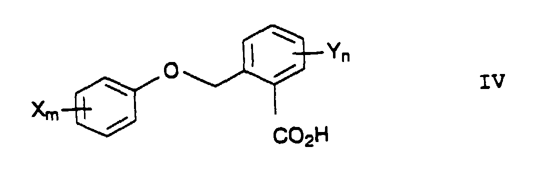 with phosgene or thionyl chloride into the corresponding o-substituted benzoyl chloride of the formula V 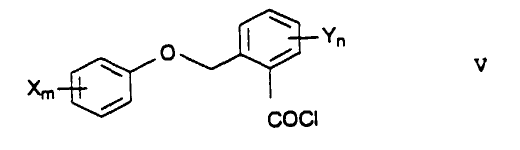 and subsequently reacting V with an alkali metal cyanide or alkaline earth metal cyanide.

2. A process as claimed in claim 1, wherein the conversion of IV into V is carried out at from 0°C to 100°C.

3. A process as claimed in claim 1, wherein the conversion of IV into V is carried out in the presence of a catalyst.

4. A process as claimed in claim 3, wherein an amide or a phosphine oxide is used as catalyst.

5. A process as claimed in claim 1, wherein the conversion of V into VI is carried out in the presence of hydrocyanic acid.

6. A process as claimed in claim 1, wherein the conversion of V into VI is carried out in a two-phase system in the presence of a phase transfer catalyst.

7. A process as claimed in claim 1, wherein the conversion of V into VI is carried out at from 0°C to the boiling point of the solvent or solvent mixture used.

8. A process as claimed in claim 1, wherein V is converted without further isolation (in situ) into VI.

9. An o-substituted benzoyl chloride of the formula V as claimed in claim 1, excepting
- 2-(phenoxymethyl)benzoyl chloride,
- 2-(3'-n-butylphenoxymethyl)benzoyl chloride,
- 2-(3'-tert-butylphenoxymethyl)benzoyl chloride,
- 2-(3',4'-dimethylphenoxymethyl)benzoyl chloride,
- 2-(4'-sec-butylphenoxymethyl)benzoyl chloride and
- 2-(4'-ethylphenoxymethyl)-4-methoxybenzoyl chloride.

10. 2-(2'-Methylphenoxymethyl)benzoyl chloride.

11. An o-substituted benzoyl cyanide of the formula VI as set forth in claim 1.

12. 2-(2'-Methylphenoxymethyl)benzoyl cyanide.

## Revendications

1. Procédé pour la préparation de cyanures de benzoyle o-substitués de formule générale VI 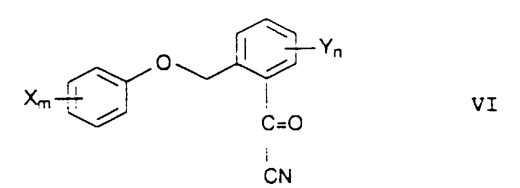 où les substituants et les indices ont la signification suivante:
X,Y halogène, alkyle en C₁-C₄, alcoxy en C₁-C₄ ou trifluorométhyle,
m un nombre entier de 0 à 4, tandis que les restes X peuvent être différents lorsque la valeur de m est supérieure à 1,
n un nombre entier de 0 à 3, tandis que les restes Y peuvent être différents lorsque la valeur de n est supérieure à 1,
caractérisé par le fait qu'on transforme un acide 2-phénoxyméthylbenzoique de formule générale IV 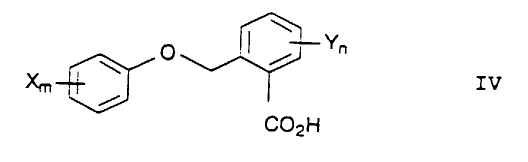 avec du phosgène ou du chlorure de thionyle en le chlorure de benzoyle o-substitué correspondant de formule générale V 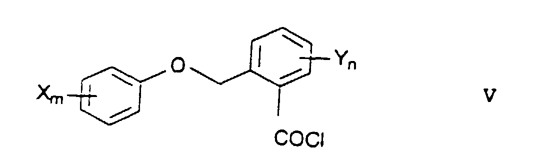 et on fait ensuite réagir V avec un cyanure de métal alcalin ou de métal alcalino-terreux.

2. Procédé selon la revendication 1, caractérisé par le fait qu'on effectue la transformation de IV en V à des températures de 0°C à 100°C.

3. Procédé selon la revendication 1, caractérisé par le fait qu'on effectue la transformation de IV en V en présence d'un catalyseur.

4. Procédé selon la revendication 3, caractérisé par le fait qu'on utilise comme catalyseur un amide ou un oxyde de phosphine.

5. Procédé selon la revendication 1, caractérisé par le fait qu'on effectue la transformation de V en VI en présence d'acide cyanhydrique.

6. Procédé selon la revendication 1, caractérisé par le fait qu'on effectue la transformation de V en VI dans un système à deux phases en présence d'un catalyseur de transfert de phases.

7. Procédé selon la revendication 1, caractérisé par le fait qu'on effectue la transformation de V en VI à des températures allant de 0°C au point d'ébullition du solvant ou mélange de solvants utilisé.

8. Procédé selon la revendication 1, caractérisé par le fait qu'on transforme V en VI sans autre isolement (in situ).

9. Chlorures de benzoyle o- substitués de formule générale V selon la revendication 1, à l'exclusion
- du chlorure d'acide 2-(phénoxyméthyl)-benzoïque,
- du chlorure d'acide 2-(3'-n-butylphénoxyméthyl)-benzoïque,
- du chlorure d'acide 2-(3'-tert-butylphénoxyméthyl)-benzoïque,
- du chlorure d'acide 2-(3',4'-diméthylphénoxyméthyl)-benzoïque,
- du chlorure d'acide 2-(4'-sec-butylphénoxyméthyl)-benzoïque et
- du chlorure d'acide 2-(4'-éthylphénoxyméthyl)-4-méthoxybenzoïque.

10. Chlorure d'acide 2-(2'-méthylphénoxyméthyl)-benzoïque.

11. Cyanures de benzoyle o- substitués de formule générale VI selon la revendication 1.

12. Cyanure de 2-(2'-méthylphénoxyméthyl)-benzoyle.
